Europäisches Patentamt

European Patent Office

Office européen des brevets

(11) Veröffentlichungsnummer: **0 270 815**
**A1**

## (12) EUROPÄISCHE PATENTANMELDUNG

(21) Anmeldenummer: 87115871.3

(22) Anmeldetag: 29.10.87

(51) Int. Cl.4: **C07C 99/00 , C07C 101/74**

(30) Priorität: 11.11.86 DE 3638364

(43) Veröffentlichungstag der Anmeldung:
15.06.88 Patentblatt 88/24

(84) Benannte Vertragsstaaten:
CH DE FR GB IT LI SE

(71) Anmelder: BAYER AG
Konzernverwaltung RP Patentabteilung
D-5090 Leverkusen 1 Bayerwerk(DE)

(72) Erfinder: Majer, Norbert, Dr.
Eibenweg 8
D-5060 Bergisch Gladbach 2(DE)
Erfinder: Backhaus, Wilhelm, Dr.
Haferkamp 8
D-5000 Köln 80(DE)

(54) Verfahren zur Herstellung von 5-aminosalicylsäure.

(57) 5-Aminosalicylsäure wird hergestellt durch Diazotierung von Anilin, anschließende Kupplung des Diazoniumsalzes auf Salicylsäure und Reduktion der erhaltenen Azoverbindung bei erhöhter Temperatur, wobei man die Reduktion unter Druck mit Alkali-, Erdalkali-und/oder Ammoniumsulfiden, -hydrogensulfiden und/oder -polysulfiden durchführt.

EP 0 270 815 A1

BAYER AKTIENGESELLSCHAFT          5090 Leverkusen, Bayerwerk

Konzernverwaltung RP

Patentabteilung                    Bg/by/bo/c

Verfahren zur Herstellung von 5-Aminosalicylsäure

Die Erfindung betrifft ein Verfahren zur Herstellung von 5-Aminosalicylsäure durch Diazotierung von Anilin, anschließende Kupplung des Diazoniumsalzes auf Salicylsäure und Reduktion der erhaltenen Azoverbindung bei erhöhter Temperatur.

Es ist bekannt, 5-Aminosalicylsäure herzustellen durch Diazotierung von Anilin in wäßriger Salzsäure, anschließende Kupplung des Diazoniumsalzes auf Salicylsäure und Reduktion der erhaltenen Azoverbindung mit Natriumhydrosulfit (Natriumdithionit) bei erhöhter Temperatur (H.E. Fierz-David und L. Blangey, Grundlegende Operationen der Farbenchemie, 8. Auflage, Wien, Springer-Verlag (1952), S. 150). Nachteilig bei dem bekannten Verfahren ist, daß es wegen des Eintragens großer Mengen von Natriumdithionit in kurzer Zeit großtechnisch kaum durchzuführen ist, da unter starker Schwefeldioxidentwicklung eine heftige exotherme Reaktion einsetzt. Darüber hinaus ist dieses Verfahren wenig wirtschaftlich

Le A 24 833-Ausland

0 270 815

- 2 -

bedingt durch die hohen Kosten des Natriumdithionits und die nicht befriedigenden Ausbeuten (etwa 80 % der Theorie an 5-Aminosalicylsäure). Außerdem ist die nach der Vorschrift von Fierz-David und Blangey erhaltene 5-Aminosalicylsäure wegen der darin noch enthaltenen Verunreinigungen, wie Salicylsäure, Anilin und Schwefel, nicht geeignet für die Verwendung als pharmazeutischer Wirkstoff.

Es wurde nun ein Verfahren zur Herstellung von 5-Aminosalicylsäure durch Diazotierung von Anilin, anschließende Kupplung des Diazoniumsalzes auf Salicylsäure und Reduktion der erhaltenen Azoverbindung bei erhöhter Temperatur gefunden, das dadurch gekennzeichnet ist, daß man die Reduktion mit Alkali-, Erdalkali- und/oder Ammoniumsulfiden, -hydrogensulfiden und/oder -polysulfiden durchführt.

Die erfindungsgemäße Reduktion der Azoverbindung wird vorteilhafterweise unter Druck durchgeführt, bevorzugt in einem Druckbereich von etwa 1 bis 6 bar absolut, besonders bevorzugt 1,5 bis 4 bar absolut, und bei Temperaturen von etwa 80 bis 180°, vorzugsweise bei 110 bis 150°C durchgeführt.

Als Alkalisulfide kommen für das erfindungsgemäße Verfahren beispielsweise Lithium-, Natrium- und Kaliumsulfid in Frage, als Erdalkalisulfide Calciumsulfid, als Alkali- oder Erdalkalipolysulfide Natrium- oder Kaliumpolysulfid, als Alkali- oder Erdalkalihydrogensulfide Natrium- oder Kaliumhydrogensulfid. Bevorzugt wird in das erfindungsgemäße Verfahren eingesetzt: Natriumhydrogensulfid, Natriumsulfid, Kaliumsulfid und/oder Ammo-

Le A 24 833

0 270 815

- 3 -

niumsulfid. Besonders bevorzugt wird in das erfindungsgemäße Verfahren Natriumhydrogensulfid eingesetzt.

Die Menge an eingesetzten Alkali-, Erdalkali- und/oder Ammoniumsulfiden, -hydrogensulfiden und/oder -polysulfiden beträgt im allgemeinen etwa 0,9 bis 5 Mol, bevorzugt 1,1 bis 2,5 Mol, pro Mol einzusetzender Salicylsäure.

Nach dem erfindungsgemäßen Verfahren wird die Reduktion in einer wäßrig-alkalischen Lösung bei einem pH-Wert von etwa 10 bis 14, bevorzugt 11 bis 12,5 durchgeführt.

Die der erfindungsgemäßen Reduktion der Azoverbindung vorausgehende Diazotierung von Anilin und anschließende Kupplung des erhaltenen Diazoniumsalzes auf Salicylsäure können nach bekannten Verfahren, z.B. wie in Fierz-David und Blangey, Grundlegende Operationen der Farbenchemie, 8. Auflage, Wien, Springer-Verlag (1952), Seite 150 beschrieben, durchgeführt werden. Dabei ist es vorteilhaft, wenn man ein Molverhältnis von Anilin zu Salicylsäure von etwa 1,5:1 bis 0,9:1, bevorzugt 1,001:1 bis 1,2:1, wählt.

Die durch Diazotierung von Anilin und anschließende Kupplung des Diazoniumsalzes auf Salicylsäure nach bekannten Verfahren erhaltene Azoverbindung wird in vorteilhafter Weise nach dem erfindungsgemäßen Verfahren ohne Zwischenisolierung direkt der Reduktion unterworfen.

Le A 24 833

0 270 815

- 4 -

Das erfindungsgemäße Verfahren kann wie folgt durchgeführt werden:

Zu einer wäßrigen, salzsauren Lösung des Diazoniumsalzes von Anilin wird unter Kühlung eine wäßrige Natriumsalicylatlösung zugegeben, die Suspension allmählich mit so viel Natronlauge versetzt, bis sich zum Schluß ein pH-Wert von etwa 12 eingestellt hat. Danach wird die erhaltene Lösung der Azoverbindung bei erhöhter Temperatur mit beispielsweise einer wäßrigen Natriumhydrogensulfidlösung versetzt, wobei sich ein Druck von etwa 2,5 bar aufbaut. Nach erfolgter Reduktion wird durch Abkühlen entspannt, das entstandene Anilin als Azeotrop mit Wasser abdestilliert, die Reaktionslösung mit Salzsäure angesäuert, die Lösung mit Aktivkohle versetzt und bei erhöhter Temperatur geklärt. Durch Kühlung der Reaktionslösung wird die 5-Aminosalicylsäure ausgefällt und anschließend isoliert.

Das erfindungsgemäße Verfahren besitzt gegenüber dem Verfahren des Standes der Technik folgende Vorteile: Es wird eine um etwa 10 % höhere Ausbeute an 5-Aminosalicylsäure erhalten. Die 5-Aminosalicylsäure enthält weniger störende Nebenprodukte und der Einsatz des teuren Natriumdithionits entfällt, wodurch das erfindungsgemäße Verfahren sich besonders wirtschaftlich gestaltet. Außerdem verkürzt sich durch den Einsatz der erfindungsgemäßen Reduktionsmittel und Reaktionsbedingungen die Reaktionszeit beträchtlich, was sich in einer gesteigerten Raum-Zeit-Ausbeute ausdrückt.

Le A 24 833

Es ist besonders überraschend, daß das erfindungsgemäße Verfahren so gute Ausbeuten an 5-Aminosalicylsäure bzw. solch gute Raum-Zeit-Ausbeuten liefert, da aus Fierz-David und Blangey, Grundlegende Operationen der Farbenchemie, 8. Auflage, Wien, Springer-Verlag (1952), Seite 152 bekannt ist, daß Natriumhydrosulfit (Natriumdithionit) die besten Resultate im Vergleich zu anderen Reduktionsmitteln ergibt.

Die 5-Aminosalicylsäure kann zur Herstellung von Azofarbstoffen verwendet werden (vgl. H.E. Fierz-David und L. Blangey, Grundlegende Operationen der Farbenchemie, 8. Auflage, Wien, Springer-Verlag (1952), Seite 150). Weiterhin dient 5-Aminosalicylsäure als Therapeutikum bei Colitis ulcerosa (vgl. Chemotherapie Telegramm 3/85).

Le A 24 833

Beispiel

In einem 8000 l fassenden emaillierten Rührwerksbehälter wurden ca. 2000 l Wasser und 350 kg (3,763 kMol) Anilin vorgelegt, 1000 l Salzsäure (30 gew.-%ig) zulaufen gelassen und mit Eis oder Sole auf -5°C gekühlt. Zu dieser Lösung wurden 787 kg einer 33 gew.-%igen Natriumnitritlösung (3,763 kMol) so zugegeben, daß die Temperatur unter +5°C blieb und am Ende der Zugabe ein kleiner Nitritüberschuß verblieb, der mit etwa 1 bis 2 kg Amidosulfonsäure zerstört wurde. Innerhalb von 2 bis 3 Stunden wurden bei 0 bis +5°C etwa 2000 l einer ca. 25 gew.-%igen Natriumsalicylatlösung (3,623 kMol) zugegeben und danach auf geringen Überschuß an diazotiertem Anilin geprüft.

Bei 0 bis 8°C wurde durch Zugabe von etwa 300 l Natronlauge (50 gew.-%ig) ein pH-Wert von 10 bis 10,5 eingestellt und die Reaktionslösung bei Raumtemperatur in einen 12500 l fassenden druckfesten Stahlkessel gedrückt. Mit Wasser wurde auf 8500 l aufgefüllt, auf 90 bis 95°C aufgeheizt, der Kessel verschlossen und 900 l Natriumhydrogensulfid-Lösung (ca. 30 gew.-%ig) zugegeben. Die Reaktionsmischung wurde im geschlossenen Kessel auf 130°C geheizt und ca. 3 Stunden bei dieser Temperatur gehalten. Dabei stellte sich ein Druck von ca. 2 bar ein.

Anschließend wurde das Reaktionsgemisch auf 90°C gekühlt, der Kessel entspannt und das entstandene Anilin azeotrop abdestilliert bis der Gehalt an Anilin kleiner

Le A 24 833

als 0,1 % betrug. Sowohl das Anilin als auch das hiervon abgetrennte Wasser können beim nächsten Ansatz wieder eingesetzt werden.

Die Reaktionsmischung wurde nun durch die Zugabe von ca. 2400 l Salzsäure (30 gew.-%ig) derart angesäuert, daß der Schwefelwasserstoff freigesetzt wurde und sich ein pH-Wert von kleiner als 0,5 einstellte. Die Ansäuerung des Reaktionsgemisches kann auch so durchgeführt werden, daß man die Salzsäure vorlegt und das Reaktionsgemisch der vorgelegten Salzsäure zugibt. Der restliche Schwefelwasserstoff und das durch Zersetzung von Thiosulfat gebildete Schwefeldioxid wurde zunächst 1 Stunde lang mit Stickstoff, danach mit Luft ausgeblasen. Das Reaktionsgemisch wurde auf 90 bis 95°C aufgeheizt, auf Sulfidgehalt geprüft und mit 50 kg Aktivkohle versetzt. Man rührte bei der eingestellten Temperatur noch einige Zeit nach, klärte dann das Reaktionsgemisch über eine Filterpresse und wusch das Filtergut anschließend mit Heißwasser nach.

Das geklärte Filtrat wurde auf ca. 50°C abgekühlt, mit Sodalösung oder Natronlauge versetzt, so daß sich ein pH-Wert von 2 bis 3 einstellte. Danach wurde auf 20 bis 25°C abgekühlt, filtriert, mit ca. 2000 l Wasser gewaschen, trockengeblasen und anschließend die ausgefallene 5-Aminosalicylsäure isoliert. Es wurden 499 kg trockenes Produkt erhalten, was einer Ausbeute von 90 % der Theorie entsprach. Analyse: 97,4 % 5-Aminosalicylsäure (aus Diazotierung), 0,14 % in Salzsäure unlösliches Produkt, <0,1 % 3-Aminosalicylsäure, 0,3 % Salicylsäure, <0,01 % Anilin. Der Rest bestand aus Kochsalz.

Le A 24 833

### Vergleichsbeispiel

Die Umsetzung wurde im Labormaßstab wie im obigen Beispiel beschrieben, durchgeführt (die jeweiligen Einsatzmengen wurden um den Faktor $2 \times 10^5$ reduziert). Die Reduktion wurde statt mit Natriumhydrogensulfid unter Druck mit Natriumdithionit drucklos durchgeführt (nach H.E. Fierz-David und L. Blangey, Grundlegende Operationen der Farbenchemie, 8. Auflage, Wien, Springer-Verlag (1952), Seite 150).

Man isolierte 5-Aminosalicylsäure in einer Menge, die einer Ausbeute von 80,2 % der Theorie entsprach. Analyse: 97,2 % 5-Aminosalicylsäure (aus Diazotierung), 0,7 % in Salzsäure unlösliches Produkt, 1 % 3-Aminosalicylsäure, 0,8 % Salicylsäure, Rest bestand aus Kochsalz.

Patentansprüche

1. Verfahren zur Herstellung von 5-Aminosalicylsäure durch Diazotierung von Anilin, anschließende Kupplung des Diazoniumsalzes auf Salicylsäure und Reduktion der erhaltenen Azoverbindung bei erhöhter Temperatur, dadurch gekennzeichnet, daß man die Reduktion mit Alkali-, Erdalkali- und/oder Ammoniumsulfiden, -hydrogensulfiden und/oder -polysulfiden durchführt.

2. Verfahren nach Anspruch 1, dadurch gekennzeichnet, daß man die Reduktion unter Druck durchführt.

3. Verfahren nach Ansprüchen 1 und 2, dadurch gekennzeichnet, daß man die Reduktion bei Drücken von 1 bis 5 bar durchführt.

4. Verfahren nach Ansprüchen 1 bis 3, dadurch gekennzeichnet, daß man die Reduktion bei Temperaturen von 80 bis 180°C durchführt.

5. Verfahren nach Ansprüchen 1 bis 4, dadurch gekennzeichnet, daß man pro Mol Salicylsäure 0,9 bis 5 Mol Alkali-, Erdalkali- und/oder Ammoniumsulfide, -hydrogensulfide und/oder -polysulfide einsetzt.

6. Verfahren nach Ansprüchen 1 bis 5, dadurch gekennzeichnet, daß man die Reduktion mit Natriumhydrogensulfid, Natriumsulfid, Kaliumsulfid und/oder Ammoniumsulfid durchführt.

Le A 24 833

7.  Verfahren nach Ansprüchen 1 bis 6, dadurch gekennzeichnet, daß man die Reduktion in einer wäßrig-alkalischen Lösung bei einem pH-Wert von 10 bis 14 durchführt.

Le A 24 833

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 104, Nr. 11, 17. März 1986, Seite 633, Zusammenfassung Nr. 88289a, Columbus, Ohio, US; & SU-A-1 159 919 (E.A. CHALYKH) 07-06-1985 ----- | 1-7 | C 07 C 99/00 C 07 C 101/74 |

RECHERCHIERTE
SACHGEBIETE (Int. Cl.4)

C 07 C 99/00
C 07 C 101/00

Der vorliegende Recherchenbericht wurde für alle Patentansprüche erstellt

| Recherchenort | Abschlußdatum der Recherche | Prüfer |
|---|---|---|
| DEN HAAG | 23-02-1988 | MOREAU J.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
                     
& : Mitglied der gleichen Patentfamilie, übereinstimmendes Dokument

EPO FORM 1503 03.82 (P0403)